(19) Europäisches Patentamt
European Patent Office
Office européen des brevets

(11) **EP 2 811 290 A1**

(12) **EUROPEAN PATENT APPLICATION**

(43) Date of publication:
**10.12.2014 Bulletin 2014/50**

(51) Int Cl.:
**G01N 27/327** (2006.01)     **G01N 33/487** (2006.01)
**A61B 5/145** (2006.01)

(21) Application number: **14171004.6**

(22) Date of filing: **03.06.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **04.06.2013   US 201313909970**

(71) Applicant: **Lifescan Scotland Limited
Inverness-shire IV2 3ED (GB)**

(72) Inventors:
• **Elder, David
  Inverness, Inverness-shire IV2 5XQ (GB)**
• **Massari, Rossano
  20126 Milano (IT)**

(74) Representative: **Brunner, John Michael Owen
Carpmaels & Ransford LLP
One Southampton Row
London WC1B 5HA (GB)**

(54) **Hand-held test meter with time-multiplexed phase detection**

(57)     A hand-held test meter for use with an analytical test strip in determining an analyte in a fluid sample includes a housing retaining a microcontroller block and a phase-shift-measurement block. The measurement block includes a signal-generation sub-block that provides an excitation voltage signal; an interface sub-block that receives the analytical test strip and permits applying the voltage excitation signal to the test strip to provide a resultant current signal; a transimpedance conversion sub-block that receives the resultant current signal and provides a resultant voltage signal; and a phase detector responsive to the resultant voltage signal and a reference signal. The microcontroller block successively provides 0°- and 90°-phase reference signals as the reference to the phase detector to measure in-phase and quadrature components and determine a phase shift corresponding to a fluid sample in the received analytical test strip.

**FIG. 3**

**Description**

**FIELD OF THE INVENTION**

The present invention relates, in general, to medical devices and, in particular, to test meters and related methods.

**DESCRIPTION OF RELATED ART**

The determination (e.g., detection or concentration measurement) of an analyte in a fluid sample is of particular interest in the medical field. For example, it can be desirable to determine glucose, ketone bodies, cholesterol, lipoproteins, triglycerides, acetaminophen or HbA1c concentrations in a sample of a bodily fluid such as urine, blood, plasma or interstitial fluid. Such determinations can be achieved using a hand-held test meter in combination with analytical test strips (e.g., electrochemical-based analytical test strips).

Hand-held and other portable test meters, e.g., for measuring blood glucose, are intended to be used repeatedly throughout the day. It is thus desirable that such meters be small and lightweight so that the user will not feel burdened while carrying one. Hand-held test meters generally operate on battery power for portability, so it is also desirable that such meters have a long battery life. Since test meters are used to make care decisions relating to medical conditions, it is desirable that they measure with as much accuracy and precision as possible.

**BRIEF DESCRIPTION OF THE DRAWINGS**

Various novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:

FIG. 1 is a simplified depiction of a hand-held test meter according to an embodiment of the present invention;

FIG. 2 is a simplified block diagram of various blocks of the hand-held test meter of FIG. 1;

FIG. 3 is a simplified block diagram of a phase-shift-measurement block and related components as can be employed in embodiments according to the present invention;

FIG. 4 is a simplified annotated schematic diagram of a dual low pass filter sub-block as can be employed in embodiments of the present invention;

FIG. 5 is a simplified annotated schematic diagram of a transimpedance conversion sub-block include a transimpedance amplifier (TIA) as can be employed in embodiments of the present invention;

FIGs. 6A-6C show a simplified annotated schematic block diagram depicting a dual low pass filter sub-block, a calibration load sub-block, an analytical test strip sample cell interface sub-block, a transimpedance conversion sub-block, an XOR phase detector and a Quadrature DEMUX phase detector as can be employed in a phase-shift-measurement block of embodiments of the present invention;

FIGs. 7-9 are flow diagrams depicting stages in a method for employing a hand-held test meter according to various embodiments of the present invention; and

FIG. 10 is a dataflow diagram of synchronous demodulation as can be employed in embodiments according to the present invention.

**DETAILED DESCRIPTION OF ILLUSTRATIVE EMBODIMENTS**

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention. As used herein, the terms "about" or "approximately" for any numerical values or ranges indicate a suitable dimensional tolerance that allows the part or collection of components to function for its intended purpose as described herein.

In general, portable test meters, such as hand-held test meters, for use with an analytical test strip in the determination of an analyte (such as glucose) in a bodily fluid sample (i.e., a whole blood sample) according to embodiments of the present invention include a housing, a microcontroller block disposed in the housing, and a phase-shift-measurement block (also referred to as a phase-shift-measurement circuit).

In such hand-held test meters, the phase-shift-measurement block includes a signal generation sub-block configured to provide an excitation voltage signal. The phase-shift-measurement block also includes an interface sub-block, e.g., for interfacing with a sample cell (also referred to herein as an "analyte chamber") in an analytical test strip. The interface block is configured to receive the analytical test strip and is electrically connected to the signal-generation block so that the voltage excitation signal is applied to the received analytical test strip to provide a resultant current signal. The phase-shift-measurement block also includes a transimpedance conversion sub-block that receives the resultant current signal and provides a resultant voltage signal. The phase-shift-measurement block also includes a phase detector responsive to the resultant voltage signal and a reference signal.

In addition, the microcontroller block is configured to successively provide (in either order) a 0°-phase reference signal and a 90°-phase reference signal as the reference signal to the phase detector to measure a phase shift corresponding to a fluid sample in the received analytical test strip, e.g., in a sample cell of an analytical test strip inserted in the hand-held test meter. The phase-shift-measurement block successively provides in-phase and quadrature components corresponding to the 0°-phase reference signal and the 90°-phase reference signal, and the microcontroller block determines the phase shift using the in-phase and quadrature components. In various embodiments, the fluid sample is a whole blood sample and the microcontroller block is further configured to compute a level of hematocrit of the fluid sample based on the determined phase shift.

Hand-held test meters according to embodiments of the present invention are beneficial in that they provide improved accuracy of analyte determination (such as glucose or hematocrit determination) in whole blood samples using simpler hardware configurations. Various embodiments can measure the hematocrit of the whole blood sample and then employ the measured hematocrit during analyte determination, e.g., glucose determination.

A problem solved by various embodiments is to accurately measure the phase of the fluid sample by successively providing 0° and 90° reference signals. Various embodiments use these reference signals to successively measure in-phase and quadrature components of the resultant voltage signal using a single phase-detecting unit. Some prior test meters instead use more than one phase-detecting unit. Although such prior meters can be useful, measuring both signal components with the same phase-detecting unit can eliminate error that can arise in due to electrical mismatch between multiple phase-detecting units. Using a single-phase detecting unit can also reduce the physical size and weight of the electronics package in the test meter compared to some prior test meters that use multiple phase-detecting units. Using a single phase-detecting unit can also reduce power consumption of the electronics, increasing battery life. The invention is not limited to solving these problems.

Once one skilled in the art is apprised of the present disclosure, he or she will recognize that an example of a hand-held test meter that can be readily modified as a hand-hand test meter according to the present invention is the commercially available OneTouch® Ultra® 2 glucose meter from LifeScan Inc. (Milpitas, California). Additional examples of hand-held test meters that can also be modified are found in U.S. Patent Application Publications Nos. 2007/0084734 (published on April 19, 2007) and 2007/0087397 (published on April 19, 2007) and in International Publication Number WO2010/049669 (published on May 6, 2010), each of which is hereby incorporated herein in full by reference.

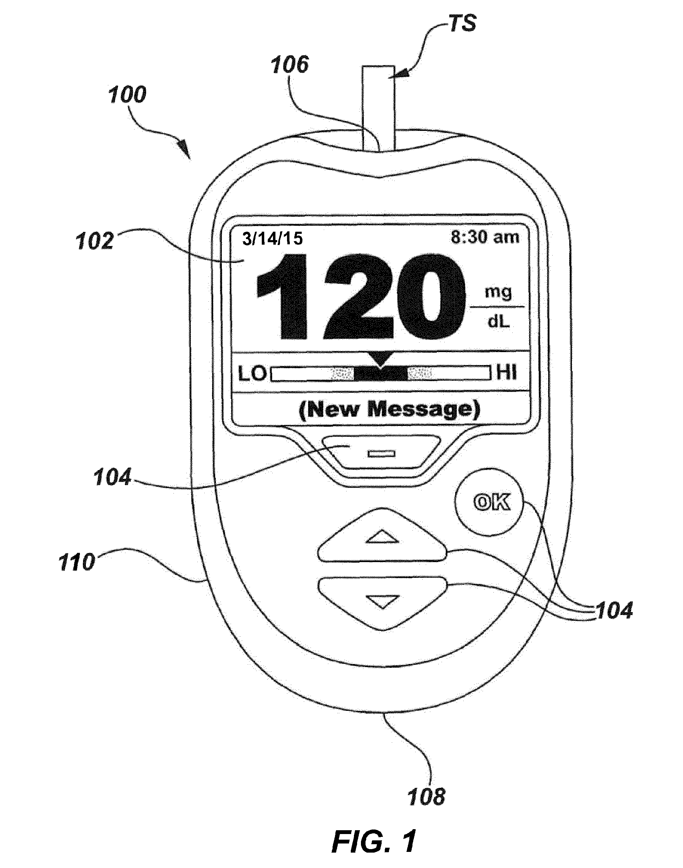

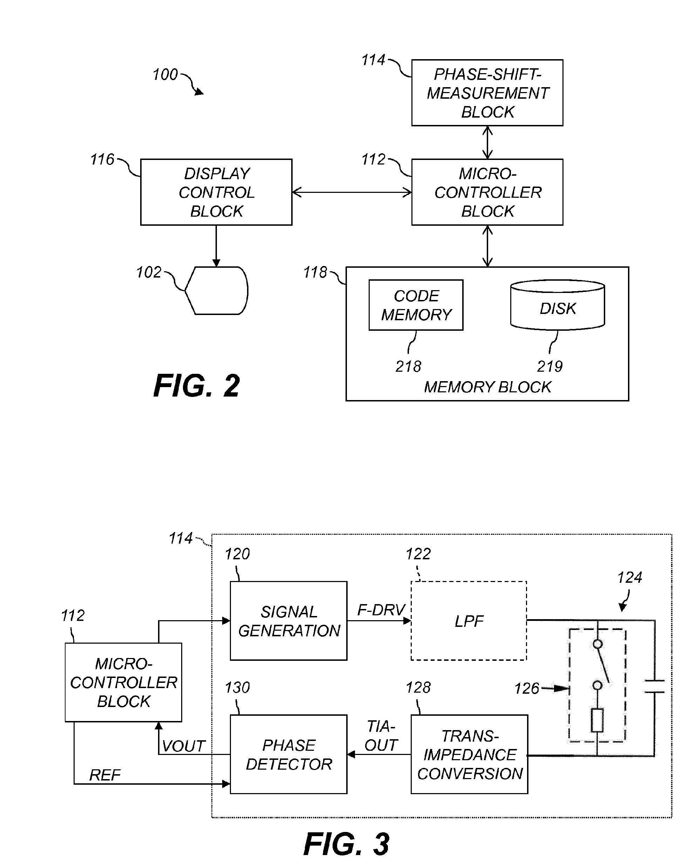

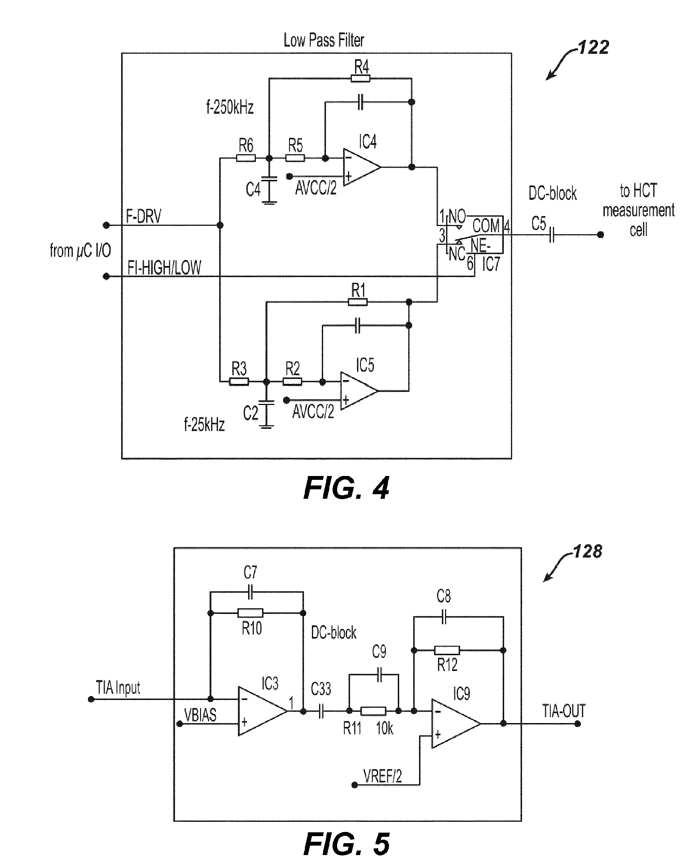

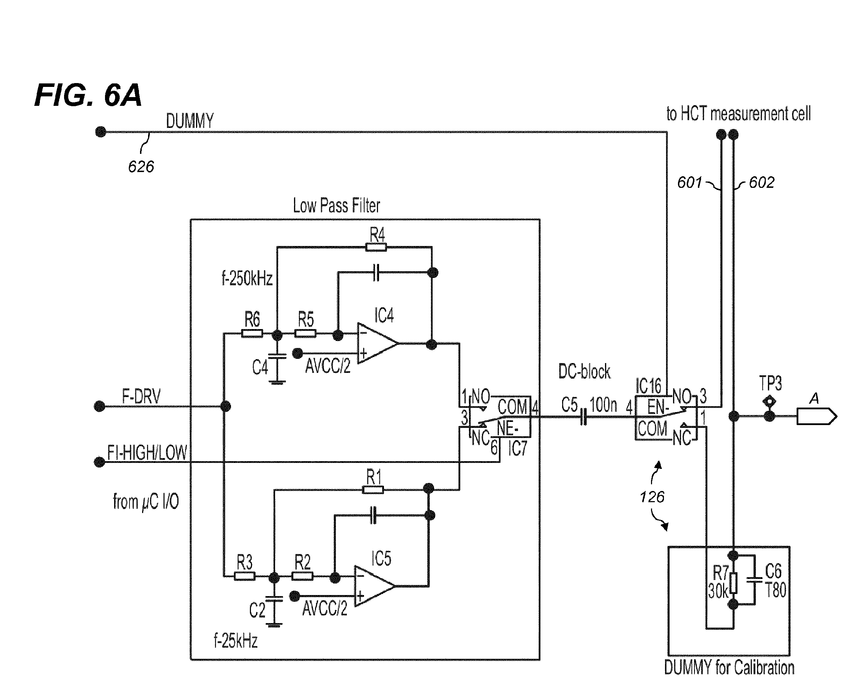

FIG. 1 is a simplified depiction of a hand-held test meter 100 according to an embodiment of the present invention. FIG. 2 is a simplified block diagram of various blocks of hand-held test meter 100. FIG. 3 is a simplified combined block diagram of a phase-shift-measurement block of hand-held test meter 100 and related components. FIG. 4 is a simplified annotated schematic diagram of a dual low pass filter sub-block of hand-held test meter 100. FIG. 5 is a simplified annotated schematic diagram of a transimpedance conversion sub-block of hand-held test meter 100. FIGs. 6A-6C show a simplified annotated schematic block diagram of portions of a phase-shift-measurement block of hand-held test meter 100.

Referring to FIGs. 1 through 6C, hand-held test meter 100 includes a display 102, a plurality of user interface buttons 104, a strip port connector 106, a USB interface 108, and a housing 110 (see FIG. 1). Referring to FIG. 2 in particular, hand-held test meter 100 also includes a microcontroller ("$\mu$C") block 112, a phase-shift-measurement block 114, a display control block 116, a memory block 118 and other electronic components (not shown) for applying a test voltage to analytical test strip (labeled TS in FIG. 1), and also for measuring an electrochemical response (e.g., plurality of test current values) and determining an analyte based on the electrochemical response. To simplify the current descriptions, the figures do not depict all such electronic circuitry.

Display 102 can be, for example, a liquid crystal display or a bi-stable display configured to show a screen image. An example of a screen image can include a glucose concentration, a date and time, an error message, and a user interface for instructing an end user how to perform a test.

Strip port connector 106 is configured to operatively interface with an analytical test strip TS, such as an electrochemical-based analytical test strip configured for the determination of glucose in a whole blood sample. Therefore, the analytical test strip is configured for operative insertion into strip port connector 106 and to operatively interface with phase-shift-measurement block 114 via, for example, suitable electrical contacts.

USB Interface 108 can be any suitable interface known to one skilled in the art. USB Interface 108 is configured to power and provide a data line to hand-held test meter 100.

Once an analytical test strip is interfaced with hand-held test meter 100, or prior thereto, a bodily fluid sample (e.g., a whole blood sample) is introduced into a sample cell of the analytical test strip. The analytical test strip can include enzymatic reagents that selectively and quantitatively transform an analyte into another predetermined chemical form. For example, the analytical test strip can be an electrochemical-based analytical test strip configured for the determination of glucose in a whole blood sample. Such a test strip can include an enzymatic reagent with ferricyanide and glucose oxidase so that glucose can be physically transformed into an oxidized form.

Memory block 118 of hand-held test meter 100 includes one or more storage device(s), e.g., code memory 218 (such as random-access memory, RAM) or disk 219 (such as a hard drive). Computer program instructions to carry out a suitable algorithm are stored in one of those device(s). Memory block 118 can also be incorporated in microcontroller block 112. Memory block 118 or microcontroller block 112 can include a Flash memory containing program firmware or a RAM for temporary variable storage). Memory block 118 can also include a Flash memory separate from microcontroller block 112 containing, e.g., graphics to be displayed on display 102, or text messages to be displayed to a user. Memory block 118 or microcontroller block 112 can also include a EEPROM or other non-volatile memory containing calibration data, user settings and algorithm parameters.

Memory block 118 can be configured, along with microcontroller block 112, to determine an analyte based on the electrochemical response of analytical test strip and the hematocrit of the introduced sample. For example, in the determination of the analyte blood glucose, the hematocrit can be used to compensate for the effect of hematocrit on electrochemically determined blood glucose concentrations.

Microcontroller block 112 is disposed within housing 110 and can include any suitable microcontroller or micro-processor known to those of skill in the art. One such suitable microcontroller is a microcontroller commercially available from Texas Instruments, Dallas, TX USA as part number MSP430F5138. This microcontroller can generate a square wave of 25 to 250kHz and a 90 degree phase-shifted wave of the same frequency and, thereby, function as a signal generation sub-block such as those described further below. The MSP430F5138 also has Analog-to-Digital (A/D) processing capabilities suitable for measuring voltages generated by phase detectors employed in embodiments of the present invention.

Throughout this description, some embodiments are described in terms that would ordinarily be implemented as software programs. Those skilled in the art will readily recognize that the equivalent of such software can also be constructed in hardware (hard-wired or programmable), firmware, or micro-code. Given the systems and methods as described herein, software or firmware not specifically shown, suggested, or described herein that is useful for implementation of any embodiment is conventional and within the ordinary skill in such arts. Microcontroller block 112 can include, e.g., a field-programmable gate array (FPGA) such as an ALTERA CYCLONE FPGA, a digital signal processor (DSP) such as a Texas Instruments TMS320C6747 DSP, or another processing device adapted to carry out algorithm(s) described herein.

Referring in particular to FIG. 3, phase-shift-measurement block 114 includes a signal generation sub-block 120, an optional low pass filter sub-block 122, an interface sub-block 124, an optional calibration load sub-block 126 (within the dashed lines of FIG. 3), a transimpedance conversion sub-block 128, and a phase detector 130.

As described further below, phase-shift-measurement block 114 and microcontroller block 112 are configured to measure a phase shift corresponding to a fluid sample in a sample cell of an analytical test strip inserted in the hand-held test meter 100. In an example, phase-shift-measurement block 114 and microcontroller block 112 are configured to measure the phase shift of one or more high frequency electrical signal(s) driven through the fluid sample (e.g., a bodily fluid sample). In various embodiments, microcontroller block 112 is further configured to compute the hematocrit of the bodily fluid based on the measured phase shift. Microcontroller block 112 can compute the hematocrit by, for example, employing an A/D converter to measure voltages received from a phase detector, convert the voltages into a phase-shift and then employing a suitable algorithm or look-up table to convert the phase-shift into a hematocrit value. Once apprised of the present disclosure, one skilled in the art will recognize that such an algorithm or look-up table will be configured to take into account various factors such as strip geometry (including electrode area and sample cell volume) and signal frequency.

It has been determined that a relationship exists between the reactance of a whole blood sample and the hematocrit of that sample. Electrical modeling of a bodily fluid sample (i.e., a whole blood sample) as parallel capacitive and resistive components indicates that when an alternating current (AC) signal is forced through the bodily fluid sample, the phase shift of the AC signal will be dependent on both the frequency of the AC voltage and the hematocrit of the sample. Moreover, modeling indicates that hematocrit has a relatively minor effect on the phase shift when the frequency of the signal is in the range of approximately 10kHz to 25kHz and a relatively significant effect on the phase shift when the frequency of the signal is in the range of approximately 250 kHz to 500kHz. Therefore, the hematocrit of a bodily fluid sample can be measured by, for example, driving AC signals of known frequency through the bodily fluid sample and detecting their phase shift. For example, phase-shift-measurement block 114 and microcontroller block 112 can be configured to measure the phase shift using the excitation voltage signal of a first frequency and a second excitation voltage signal of a second frequency. The phase shift of a signal with a frequency in the range of 10kHz to 25kHz can be used as a reference reading in such a hematocrit measurement, e.g., of a whole blood sample, while the phase shift of a signal with a frequency in the range of 250 kHz to 500kHz can be used as the primary measurement. The phase shift of a signal with a frequency of about 75kHz or higher, or of about 75kHz to about 500kHz, can also be used as the primary measurement.

Referring to FIGs. 3 through 6C in particular, signal generation sub-block 120 can be any suitable signal generation block and is configured to generate an excitation voltage signal (F-DRV). The excitation voltage signal can be, e.g., a square wave (e.g., swinging between 0V and a reference voltage Vref) or a sinusoidal wave of a desired frequency. Such a signal generation sub-block can, if desired, be integrated into microcontroller block 112.

The excitation voltage signal generated by signal generation sub-block 120 can be communicated directly to interface sub-block 124, or to optional dual low pass filter (LPF) sub-block 122, which is configured to convert a square wave excitation voltage signal to a sine wave signal of a predetermined frequency. Low pass filter sub-block 122 can filter out the odd harmonics in a square wave, leaving the fundamental. Low pass filter sub-block 122 can, if desired, be integrated into signal-generation sub-block 120.

FIG. 4 shows an exemplary dual low-pass filter sub-block 122 configured to provide both a signal of a first frequency (such as a frequency in the range of 10kHz to 25kHz) and a signal of a second frequency (such as a frequency in the range of 250 kHz to 500kHz) to the analytical test strip sample cell interface sub-block and an analytical test strip's sample cell (also referred to as the HCT measurement cell). Selection of the first and second frequency is accomplished using switch IC7 of FIG. 4. The dual LPF of FIG. 4 includes employs two suitable operational amplifiers (IC4 and IC5) such as the operational amplifier available from Texas Instruments, Dallas, Texas, USA as high-speed, voltage feedback, CMOS operational amplifier part number OPA354.

Referring to FIG. 4, F-DRV represents a square wave input of either a low or high frequency (e.g., 25kHz or 250 kHz) and is connected to both IC4 and IC5. Signal FI-HIGH/LOW (from the microcontroller block 112) selects the output of dual low pass filter sub-block 122 via switch IC7. C5 in FIG. 4 is configured to block the operating voltage of dual low pass filter sub-block 122 from the HCT measurement cell. These signals can also be used to control the low-pass filter shown in FIG. 6A.

Although a specific dual LPF is depicted in FIG. 4, dual low pass filter sub-block 122 can be any suitable low pass filter sub-block known to one skilled in the art including, for example, any suitable multiple feedback low pass filter, or a Sallen and Key low pass filter.

Referring back to FIG. 3, the sine wave produced by low pass filter sub-block 122 (or other excitation voltage signal produced by the signal-generation sub-block 120) is communicated to interface sub-block 124 electrically connected to signal-generation sub-block 120. In interface sub-block 124, the excitation voltage signal is applied to the received analytical test strip, e.g., driven across the sample cell of the analytical test strip (also referred to as an HCT measurement cell). A resultant current signal is produced, as discussed below.

FIG. 6A also depicts a calibration load sub-block 126 that includes a switch (IC16) and a dummy load R7 and C6. Calibration load sub-block 126 is configured for the dynamic measurement of a phase offset for the known phase shift of zero degrees produced by resistor R7, thus providing a phase offset for use in calibration. C6 is configured to force a predetermined slight phase shift, e.g., to compensate for phase delays caused by parasitic capacities in the signal traces to the sample cell. Microcontroller block 112 determines whether the excitation voltage is applied to the analytical test strip or to the calibration load sub-block 126 by setting the value of control input 626 ("DUMMY").

Interface sub-block 124 can be any suitable interface block including, for example, a strip port connector (SPC) configured to operatively interface with the sample cell of the analytical test strip via first electrode and second electrodes of the analytical test strip disposed at least partly in the sample cell of the analytical test strip. In such a configuration, the signal can be driven into the sample cell (from the low pass filter sub-block 122 or signal generation sub-block 120) via the first electrode 601 and picked-up from the sample cell (by the transimpedance conversion sub-block 128) via the second electrode 602 as depicted in FIG. 6A (at "to HCT measurement cell").

The resultant current produced by driving the signal across the sample cell is picked-up by transimpedance conversion sub-block 128 and converted into a resultant voltage signal for communication to phase detector 130. Phase detector 130 is responsive to the resultant voltage signal and a reference signal.

Transimpedance conversion sub-block 128 can be any suitable transimpedance sub-block known to one skilled in the art. FIG. 5 is a simplified annotated schematic block diagram of a transimpedance conversion sub-block 128 including a transimpedance amplifier (TIA). The example shown is based on two OPA354 operational amplifiers, IC3 and IC9. The input ("TIA Input") is the resultant current signal. The first stage of TIA sub-block 128 operates at, for example, 400mV, which limits the output AC amplitude to +/-400mV. The second stage of TIA sub-block 128 operates at Vref/2, a configuration which enables the generation of an output of the full span of the microcontroller A/D inputs. C9 of TIA sub-block 128 serves as a blocking component that only allows an AC (e.g., sine wave) signal to pass. The output of the transimpedance conversion sub-block 128 is signal TIA-OUT, which is the resultant voltage signal.

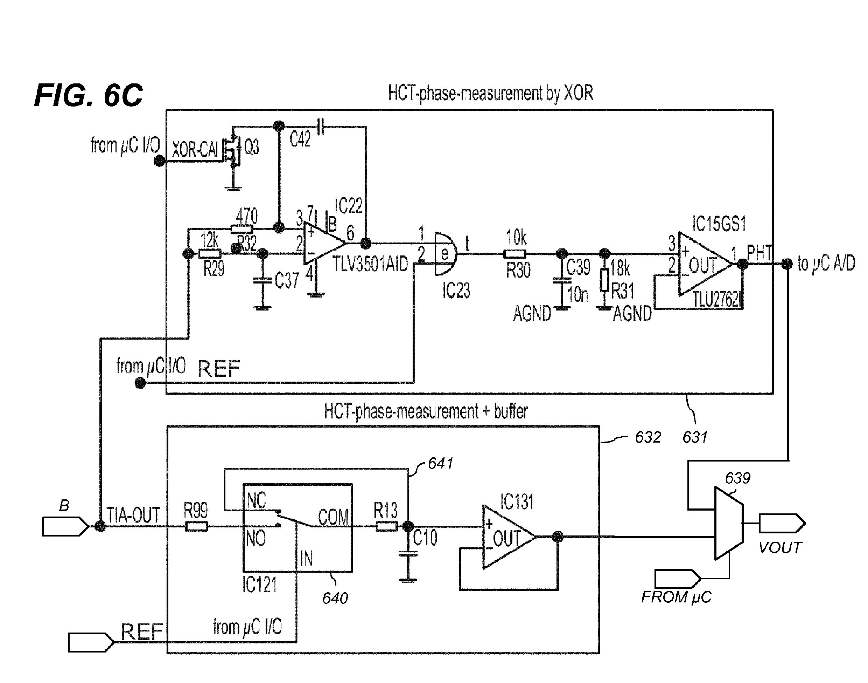

Phase detector 130 can be any suitable phase detector that produces either a digital frequency that can be read back by microcontroller block 112 using a capture function, or an analog voltage that can be read back by microcontroller block 112 using an analog to digital converter. FIGs. 6A-6C depict a schematic that includes two such phase detectors, namely an XOR phase detector 631 (in the upper half of FIG. 6C and including IC22 and IC23) and a Quadrature DEMUX phase detector 632 (in the lower half of FIG. 6C and including IC12 and IC13). Referring back to FIG. 3, phase detector 130 is responsive to resultant voltage signal TIA-OUT from transimpedance conversion sub-block 128 and reference signal REF from microcontroller block 112.

Phase detector 130 produces voltage output signal VOUT corresponding to the phase of TIA-OUT with respect to REF. In an example, microcontroller block 112 is configured to provide square waves as two successive signals on REF: a 0°-phase reference signal and a 90°-phase reference signal. Phase detector 130 can include or be followed by buffers or sample-and-hold units to retain successively-produced VOUT signals corresponding to the 0°-phase reference signal and to the 90°-phase reference signal. An example of a phase detector 130 is shown in FIG. 6C.

FIG. 6C shows Quadrature DEMUX phase detector 632 including a switch (IC121), a filter (R13 and C10), and a buffer (IC131). These components are used to successively measure the resistive and reactive portions of the AC signal from transimpedance conversion sub-block 128. Measuring both resistive and reactive portions advantageously provides a measurement range of phase that covers 0 degrees to 360 degrees. The Quadrature DEMUX phase detector 632 of FIG. 6C generates an output voltage corresponding to the phase difference between TIA-OUT (the resultant voltage signal) and REF (the reference signal).

The term "quadrature" refers to the fact that Quadrature DEMUX phase detector 632 can demodulate a signal based on two orthogonal reference frequencies, e.g., 0° and 90°. In this example, one signal-processing path is used for both reference frequencies in turn. Quadrature DEMUX circuits are also referred to as "synchronous demodulators" or "lock-in amplifiers." Further details of lock-in amplifiers are provided in "Lock-in Amplifiers" published by Bentham Instruments Ltd. as document 225.02 (2010), the disclosure of which is incorporated herein by reference. Pages 7 and 8 of this document show an example of a quadrature demux (lock-in amplifier) and how it acts as a multiplier. Quadrature DEMUX phase detector 632 is an example of a synchronous-demodulation phase-detecting unit.

F-DRV is the voltage excitation signal; shown in FIG. 6A. When REF is substantially in phase with F-DRV, i.e., the 0°-phase reference signal is provided as REF, the output voltage VOUT represents the "in phase measurement" and is proportional to the "resistive" part of the AC signal. When REF is substantially 90° out of phase with F-DRV, the output voltage VOUT represents the "Quadrature Measurement" and is proportional to the "reactive" part of the signal. The phase shift is calculated as:

$$\Phi = \tan^{-1}(V_{QUAD-PHASE} / V_{IN-PHASE})$$

or

$$\Phi = \text{atan2}(V_{IN-PHASE}, V_{QUAD-PHASE})$$

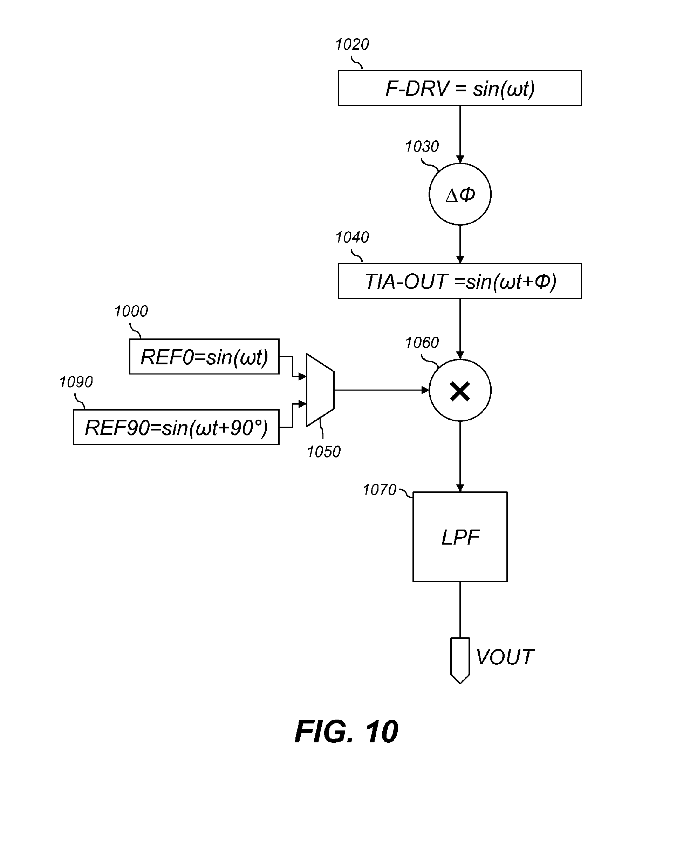

where atan2(x, y) is the four-quadrant arc-tangent of *y/x.* Note that the 90°-phase signal can lead or lag the 0°-phase signal, and the 0°-phase signal can also be 180° out of phase with the voltage excitation signal. FIG. 10, discussed below, shows an exemplary dataflow of quadrature demodulation (DEMUX).

Such a Quadrature DEMUX phase detector circuit can also be employed to measure the impedance of a bodily fluid sample in the sample cell. It is hypothesized, without being bound, that the impedance could be employed along with the phase shift, or independently thereof, to determine the hematocrit of the bodily sample. The amplitude (magnitude) of a signal forced through the sample cell can be calculated using the two voltage outputs of the Quadrature DEMUX circuit as follows:

$$\text{Amplitude} = ((V_{\text{QUAD-PHASE}})^2 + (V_{\text{IN-PHASE}})^2)^{0.5}$$

This amplitude can then be compared to an amplitude measured for the known resistor of calibration load sub-block 126 to determine the impedance.

The XOR phase detector 631 has a measurement range of 0° to 180°, or alternatively a measurement range of -90° to +90°, depending whether the REF input from microcontroller block 112, e.g., a square wave, is in phase to the F-DRV sine wave or is set to a 90° phase shift. The XOR phase detector produces an output frequency that is always double the input frequency, however the duty cycle varies. If both inputs are perfectly in phase, the output is LOW, if both inputs are 180° shifted the output is always HIGH. By integrating the output signal (e.g. via a simple RC element) a voltage can be generated that is directly proportional to the phase shift between both inputs.

Microcontroller block 112 and phase-shift-measurement block 114 are configured to cooperate to perform a sequential measurement of real (in-phase) and imaginary (quadrature) components of an input signal. In various embodiments, microcontroller block 112 is configured to successively provide a 0°-phase reference signal REF and a 90°-phase reference signal REF as the reference signal to the phase detector 130 (e.g., to phase detector 631 or phase detector 632). The phase-shift-measurement-block 114 thus successively measures in-phase and quadrature components of the resultant voltage signal. Microcontroller block 112 receives the corresponding output voltage(s) VOUT from one of the phase detector(s), e.g., through multiplexer 639 controlled by microcontroller block 112. Microcontroller block 112 is further configured to use the measured $V_{\text{QUAD-PHASE}}$ and $V_{\text{IN-PHASE}}$ signals to determine a phase shift of a fluid sample in the received analytical test strip, as described above (e.g., using atan2), and can be configured to use those signals to determine a fluid-sample resultant-voltage-signal magnitude as discussed above.

Microcontroller block 112 can be configured so that, after beginning to provide each reference signal, microcontroller block 112 continues providing that reference signal for a selected length of time before reading VOUT. This can provide time for the components of phase detector 130 to stabilize. For example, the reference signal can be provided for 10ms to permit a 10KΩ / 100nF low-pass filter (time constant ~1 ms) to stabilize. In various embodiments, an A/D converter takes 1-10μs to read VOUT and provide a corresponding digital signal. Microcontroller block 112 continues to provide the appropriate reference signal during that time.

Hand-held test meter 100 can include only one phase detector 130, e.g., Quadrature DEMUX phase detector 632. In various embodiments, the phase detector 130 includes a single phase-detecting unit 640 configured to detect a phase of the resultant voltage signal (TIA-OUT) with respect to the reference signal (REF). Using only one phase-detecting unit instead of two can advantageously permit constructing a smaller, lighter hand-held test meter 100 and increasing battery life of hand-held test meter 100. In Quadrature DEMUX phase detector 632, the phase-detecting unit 640 is a switch. Phase-detecting unit 640 can also be a field-effect transistor (FET) biased to perform a switching function. In phase detector 631, the phase-detecting unit is XOR gate IC23. The phase-detecting unit can also be an edge detector.

Referring to FIG. 6C, in various embodiments, phase-detecting unit 640 is a switch with a control input (IN), a common terminal (COM), a normally-closed terminal (NC), and a normally-open terminal (NO). TIA-OUT is connected to the NC terminal and a low-pass filter (R13 and C10) is connected to the COM terminal. In another example, COM is connected to R99 and NO is connected to R13. R13 can be a 0-ohm shunt. In an example, R99 is a 0-ohm shunt and R13 and C10 form the low-pass filter. In another example, R13 is a 0-ohm shunt, and R99 and C10 form the low-pass filter.

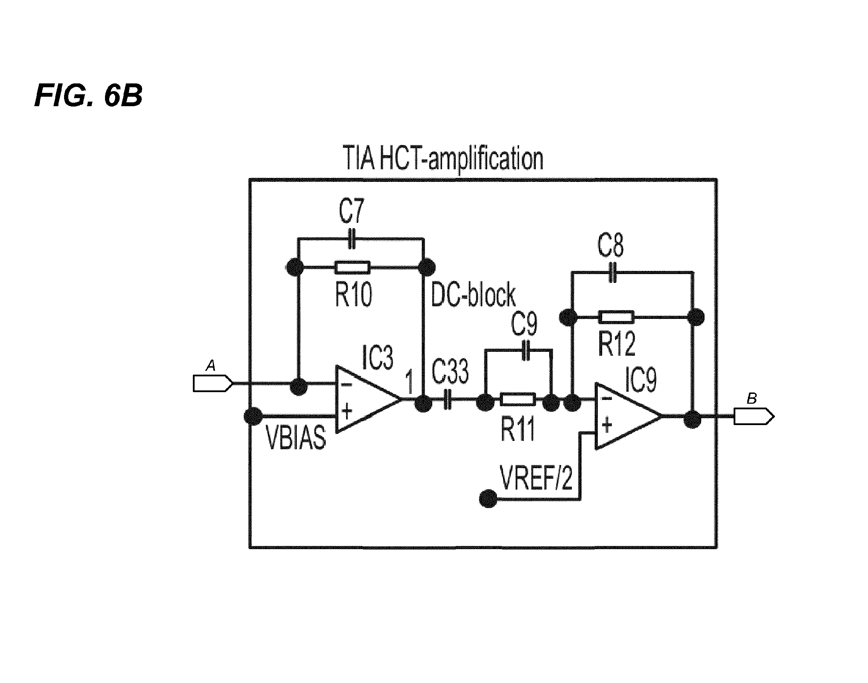

In various embodiments, conductor 641 connects the NC terminal to the node between R13 and C10. In other embodiments, conductor 641 connects the NC terminal to the COM terminal. Conductor 641 introduces the parasitic capacitance of the NC terminal to the input of buffer IC131, and thus to the output of the Quadrature DEMUX phase detector 632. Therefore, the capacitance seen at the COM terminal (the switch output) remains relatively constant whether the switch (phase-detecting unit 640) is open or closed. Without conductor 641, TIA-OUT has to charge a different capacitance depending on whether the switch is open or closed. This can introduce error, particularly in examples in which TIA-OUT is AC-coupled (e.g., a blocking capacitor is placed in series between IC9, FIG. 6B, and R99, FIG. 6C). Conductor 641 can reduce the probability or severity of measurement inaccuracies due to this error.

In various embodiments of the present invention, phase-detecting unit 640 is a switch having a common terminal, a pass-through terminal (e.g., NO in FIG. 6C), and an open-circuit terminal (e.g., NC). The switch selectively conveys signals between electronics attached to the common terminal (e.g., R13 or C10) and electronics attached to the pass-through terminal (e.g., R99). Conductor 641 electrically connects the open-circuit terminal and the common terminal. The term "open-circuit" is used in this context solely to identify the terminal not involved in conveying signals between electronics. Conductor 641 or parasitics may cause the "open-circuit terminal" not to be electrically disconnected from all other electronic components in the hand-held test meter.

Once apprised of the present disclosure, one skilled in the art will recognize that phase detector sub-blocks employed in embodiments of the present invention can take any suitable form and include, for example, forms that employ rising edge capture techniques, dual edge capture techniques, XOR techniques and synchronous demodulation techniques.

Since low pass filter sub-block 122, transimpedance conversion sub-block 128 and phase detector 130 can introduce a residual phase shift into phase-shift-measurement block 114, calibration load sub-block 126 can be optionally included in the phase-shift-measurement block. Calibration load sub-block 126 is configured to be essentially resistive in nature (for example a 33k-ohm load) and, therefore, induces no phase shift between excitation voltage and generated current. Calibration load sub-block 126 is configured to be switched in across the circuit to give a "zero" calibration reading. Once calibrated, the hand-held test meter can measure the phase shift of a bodily fluid sample, subtract the "zero" reading to compute a corrected phase shift and subsequently compute the bodily sample hematocrit based on the corrected phase shift.

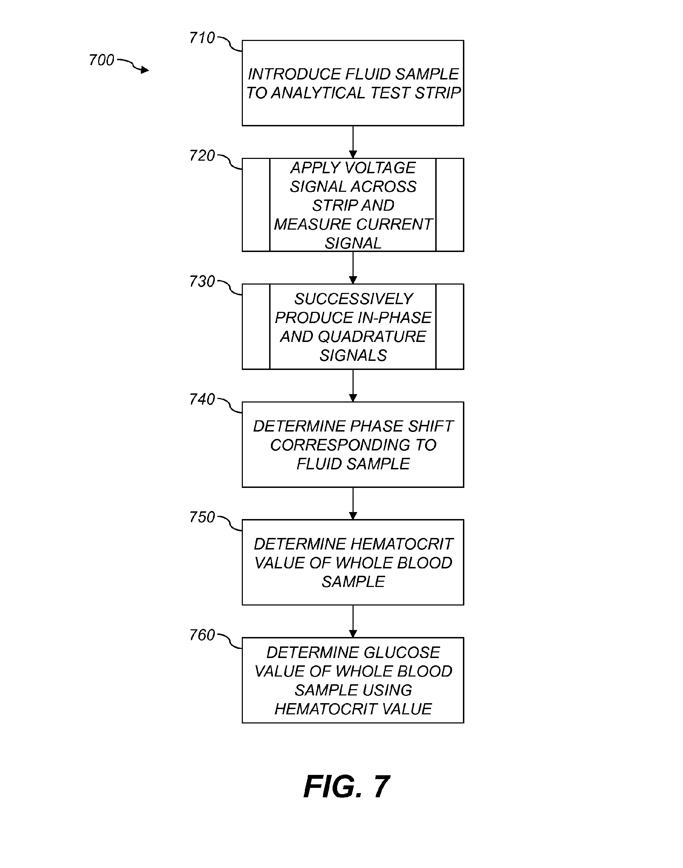

FIG. 7 is a flow diagram depicting stages in a method 700 for employing a hand-held test meter and analytical test strip (e.g., an electrochemical-based analytical test strip). Reference is made to various components described above for exemplary purposes. Methods described herein are not limited to being performed only by the identified components.

Method 700, at step 710, includes introducing a fluid sample, e.g., a whole blood sample to the analytical test strip (e.g., into a sample cell of the analytical test strip).

At step 720, a voltage signal is applied across the test strip (e.g., across the sample cell). A resultant current signal is measured.

At step 730, in-phase and quadrature signals are successively produced using the measured resultant current signal. The signals can be produced by a phase-shift-measurement block of a hand-held test meter. This can be performed as described above. In an example, microcontroller block 112 drives REF with a 0° reference signal, then measures VOUT ($V_{IN-PHASE}$), then drives REF with a 90° reference signal, and then measures VOUT ($V_{QUAD-PHASE}$) again. The quadrature signal can be produced before or after the in-phase signal. An exemplary dataflow of this step is discussed below with reference to FIG. 10.

At step 740, a phase shift corresponding to the fluid sample applied to the test strip is automatically determined using a microcontroller block of a hand-held test meter. This can be done as described above using the atan2 function. In various embodiments, an impedance magnitude of the fluid sample applied to the test strip is also automatically determined in step 740 using the phase-shift-measurement block of the hand-held test meter based on the in-phase and quadrature signals. This can be done using the amplitude computation described above.

At step 740, in embodiments in which the fluid sample is a whole blood sample, method 700 can further include computing a hematocrit value of the whole blood sample based on the determined phase shift using the microcontroller block.

Once apprised of the present disclosure, one skilled in the art will recognize that methods according to embodiments of the present invention, including method 700, can be readily modified to incorporate any of the techniques, benefits and characteristics of hand-held test meters according to embodiments of the present invention and described herein. For example, if desired, an analyte in the introduced bodily fluid sample can be determined using the analytical test strip, hand-held test meter and computed hematocrit. For example, at step 760, glucose is automatically determined using the microcontroller block in response to the determined hematocrit. Step 760 can include determining an estimated glucose value, then adjusting the estimated glucose value using the computed hematocrit to determine a more accurate glucose value. In this example, the analytical test strip is an electrochemical-based analytical test strip configured for the determination of glucose in a whole blood sample.

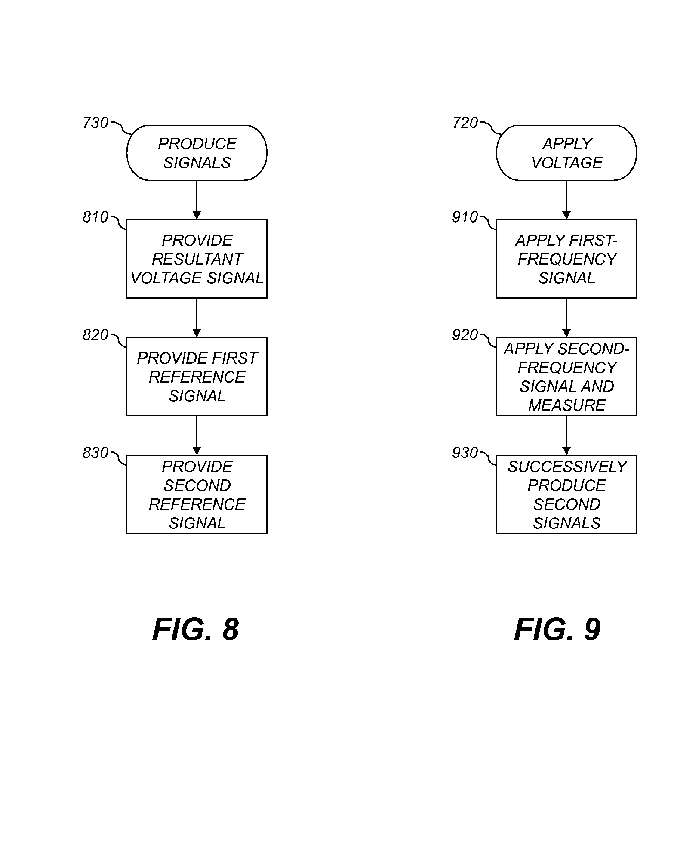

FIG. 8 is a flow diagram depicting stages in method 700, and specifically stages in step 730 of producing the in-phase and quadrature signals. At step 810, a resultant voltage signal corresponding to the resultant current signal is provided to a phase detector of the hand-held test meter, e.g., to phase detector 130, FIG. 3. The phase detector can include a single phase-detecting unit configured to detect a phase of the resultant voltage signal with respect to the provided reference signal. The phase-detecting unit can be, e.g., a rising-edge-capture phase-detecting unit, a synchronous-demodulation phase-detecting unit, a dual-edge-capture phase-detecting unit, or an XOR-based phase-detecting unit.

At step 820, a first reference signal having a first phase is provided to the phase detector. For example, the first phase can be 0°.

At step 830, subsequent to step 820, a second reference signal is provided to the phase detector. The second signal has a second phase 90° apart from the first phase. For example, the second phase can be 90°. If the first phase is 90°, the second phase can be 0°. The first and second reference signals can be square waves, and can be provided using the microcontroller block.

FIG. 9 is a flow diagram depicting stages in method 700, and specifically stages in step 720 of applying the voltage signal. At step 910, the voltage signal is applied at a first frequency. At step 920, a second voltage signal is applied across the test strip at a second frequency different from the first frequency and a second resultant current signal is measured. At step 930, second in-phase and second quadrature signals are successively produced using the measured second resultant current signal. In these embodiments, the determining-phase-shift step 740, FIG. 7, includes determining the phase shift based on the in-phase, quadrature, second in-phase, and second quadrature signals. The first frequency can be, e.g., in the range of about 10kHz to about 25kHz and the second frequency can be in the range of about 250kHz to about 500kHz.

FIG. 10 shows a dataflow diagram of an example of synchronous demodulation, e.g., as used in quadrature DEMUX phase detector 632, FIG. 6C. Excitation voltage signal 1020 (F-DRV) is of the form sin(ωt). Phase shift 1030 (ΔΦ) represents the shift in phase through calibration load sub-block 126 or through the sample cell of the analytical test strip. Resultant voltage signal 1040 (TIA-OUT) is one operand of multiplication 1060, and is of the form sin(ωt+Φ). As indicated by multiplexer 1050, either 0°-phase signal 1000 (REF0) or 90°-phase signal 1090 (REF90) is the other operand of multiplication 1060.

In an example in which REF0 is multiplied by TIA-OUT, the product from multiplication 1060 is an intermediate signal

$$0.5\cos(\Phi) - 0.5\cos(2\omega t+\Phi).$$

This signal is filtered by the low-pass filter 1070 to retain substantially only the DC component. That is, the $\cos(2\omega t+\Phi)$ term is removed from the intermediate signal, leaving only a DC signal (VOUT) with the value

$$0.5\cos(\Phi).$$

This is a DC (substantially non-time-varying) value since it does not depend on the value of t. This DC component is an in-phase (or "real") component I. Similarly, in an example in which REF90 is multiplied by TIA-OUT, the resulting DC component after low-pass filtering is a quadrature (or "imaginary") component Q. In either example, TIA-OUT is preferably a signal with substantially only one sinusoidal frequency component. This reduces the probability of other frequencies being introduced into the DC component.

**PARTS LIST FOR FIGS. 1-10**

| | |
|---|---|
| 100 | hand-held test meter |
| 102 | display |
| 104 | user interface buttons |
| 106 | strip port connector |
| 108 | USB Interface |
| 110 | housing |
| 112 | microcontroller block |
| 114 | phase-shift-measurement block |
| 116 | display control block |
| 118 | memory block |
| 120 | signal generation sub-block |
| 122 | low pass filter sub-block |
| 124 | interface sub-block |
| 126 | calibration load sub-block |
| 128 | transimpedance conversion sub-block |
| 130 | phase detector |
| 218 | code memory |
| 219 | disk |
| 601 | first electrode |
| 602 | second electrode |
| 626 | control input |
| 631, 632 | phase detectors |
| 639 | multiplexer |
| 640 | phase-detecting unit |
| 641 | conductor |

| 700 | method |
| 710, 720, 730 | steps |
| 740, 750, 760 | steps |
| 810, 820, 830 | steps |
| 910,920,930 | steps |
| 1000 | 0°-phase signal |
| 1020 | excitation voltage signal |
| 1030 | phase shift |
| 1040 | resultant voltage signal |
| 1050 | multiplexer |
| 1060 | multiplication |
| 1070 | low-pass filter |
| 1090 | 90°-phase signal |

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein can be employed in practicing the invention. References to "a particular embodiment" and the like refer to features that are present in at least one embodiment of the invention. Separate references to "an embodiment" or "particular embodiments" or the like do not necessarily refer to the same embodiment or embodiments; however, such embodiments are not mutually exclusive, unless so indicated or as are readily apparent to one of skill in the art. The word "or" is used in this disclosure in a non-exclusive sense, unless otherwise explicitly noted. It is intended that the following claims define the scope of the invention and that devices and methods within the scope of these claims and their equivalents be covered thereby.

**Claims**

1. A hand-held test meter for use with an analytical test strip in the determination of an analyte in a fluid sample, the test meter comprising:

   a housing;
   a microcontroller block disposed in the housing;
   a phase-shift-measurement block that includes:

      a signal-generation sub-block configured to provide an excitation voltage signal;
      an interface sub-block configured to receive the analytical test strip, the interface sub-block electrically connected to the signal-generation sub-block so that the voltage excitation signal is applied to the received analytical test strip to provide a resultant current signal;
      a transimpedance conversion sub-block that receives the resultant current signal and provides a resultant voltage signal; and
      a phase detector responsive to the resultant voltage signal and a reference signal;

   wherein the microcontroller block is configured to successively provide a 0°-phase reference signal and a 90°-phase reference signal as the reference signal to the phase detector so that the phase-shift-measurement-block successively measures in-phase and quadrature components of the resultant voltage signal and the microcontroller block determines a phase shift corresponding to the fluid sample in the received analytical test strip using the measured in-phase and quadrature components.

2. The test meter of claim 1, wherein the fluid sample is a whole blood sample and the microcontroller block is further configured to compute a level of hematocrit of the fluid sample based on the determined phase shift.

3. The test meter of claim 1, wherein the phase detector includes a single phase-detecting unit configured to detect a phase of the resultant voltage signal with respect to the reference signal.

4. The test meter of claim 3, wherein the phase-detecting unit is a switch.

5. The test meter of claim 4, wherein the phase detector further includes a conductor connecting a common terminal of the switch to an open-circuit terminal of the switch.

6. The test meter of claim 4, wherein the phase-detecting unit is one of an exclusive-OR gate and an edge detector.

7. The test meter of claim 1, wherein the microcontroller block is configured to provide square waves as the 0°-phase reference signal and the 90°-phase reference signal.

8. The test meter of claim 1, wherein the phase-shift-measurement block and the microcontroller block are configured to determine the phase shift using the excitation voltage signal of a first frequency and a second excitation voltage signal of a second frequency.

9. The test meter of claim 8, wherein the fluid sample is a whole blood sample and wherein the first frequency is in the range of about 10kHz to about 25kHz and the second frequency is in the range of about 75kHz to about 500kHz.

10. The test meter of claim 1, wherein the interface sub-block is configured to operatively interface with the received analytical test strip via a first electrode and a second electrode of the received analytical test strip, the first and second electrodes disposed at least partly in a sample cell of the received analytical test strip.

11. The test meter of claim 1, wherein the analytical test strip is an electrochemical-based analytical test strip configured for the determination of glucose in a whole blood sample.

12. A method for employing a hand-held test meter and an analytical test strip, the method comprising:

introducing a fluid sample to the analytical test strip;
applying a voltage signal across the test strip and measuring a resultant current signal;
successively producing in-phase and quadrature signals using the measured resultant current signal; and
automatically determining a phase shift corresponding to the fluid sample applied to the test strip using a phase-shift-measurement block of the hand-held test meter based on the in-phase and quadrature signals.

13. The method of claim 12, wherein the fluid sample is a whole blood sample, the method further including automatically computing a hematocrit value of the whole blood sample based on the determined phase shift using a microcontroller block of the test meter.

14. The method of claim 12, wherein the analytical test strip is an electrochemical-based analytical test strip configured for the determination of glucose in a whole blood sample.

15. The method of claim 12, wherein the step of producing the in-phase and quadrature signals includes:

providing a resultant voltage signal corresponding to the resultant current signal to a phase detector of the hand-held test meter;
providing a first reference signal having a first phase to the phase detector; and
subsequently providing to the phase detector a second reference signal having a second phase 90° apart from the first phase.

16. The method of claim 15, wherein the providing-reference-signal steps include providing respective square waves as the 0°-phase reference signal and the 90°-phase reference signal using a microcontroller block of the hand-held test meter.

17. The method of claim 15, wherein the phase detector includes a single phase-detecting unit configured to detect a phase of the resultant voltage signal with respect to the provided reference signal.

18. The method of claim 17, wherein the phase-detecting unit is a rising-edge-capture phase-detecting unit.

19. The method of claim 17, wherein the phase-detecting unit is a synchronous-demodulation phase-detecting unit.

20. The method of claim 17, wherein the phase-detecting unit is one of a dual-edge-capture phase-detecting unit and an XOR-based phase-detecting unit.

21. The method of claim 12, wherein the applying-voltage step includes applying the voltage signal at a first frequency, the method further including applying a second voltage signal across the test strip at a second frequency different from the first frequency and measuring a second resultant current signal, and successively producing second in-phase and second quadrature signals using the measured second resultant current signal, wherein the determining-phase-shift step includes determining the phase shift based on the in-phase, quadrature, second in-phase, and second quadrature signals.

22. The method of claim 21, wherein the first frequency is in the range of about 10kHz to about 25kHz and the second frequency is in the range of about 75kHz to about 500kHz.

23. The method of claim 12, further including automatically determining an impedance magnitude of the fluid sample applied to the test strip using the phase-shift-measurement block of the hand-held test meter based on the in-phase and quadrature signals.

**FIG. 1**

100

114 ── PHASE-SHIFT-
MEASUREMENT
BLOCK

116 ── DISPLAY
CONTROL
BLOCK

112 ── MICRO-
CONTROLLER
BLOCK

102

118 ── MEMORY BLOCK

CODE
MEMORY

DISK

218            219

## FIG. 2

114 ──

112            120            122            124

MICRO-
CONTROLLER
BLOCK

SIGNAL
GENERATION

F-DRV            LPF

126 ──

130            128

VOUT            PHASE
DETECTOR

TIA-
OUT            TRANS-
IMPEDANCE
CONVERSION

REF

## FIG. 3

**FIG. 4**

**FIG. 5**

# FIG. 6A

DUMMY

to HCT measurement cell

626

601  602

Low Pass Filter

R4

f-250kHz

R6  R5  IC4

C4  AVCC/2

F-DRV

DC-block

IC16

TP3

A

1 NO COM 4
3 NC NE-
6 IC7

C5  100n  4  EN-  NO 3
COM  NC 1

FI-HIGH/LOW

126

from µC I/O

R1

IC5

R3  R2

C2  AVCC/2

f-25kHz

R7
30k

C6
T80

DUMMY for Calibration

FIG. 6B

TIA HCT-amplification

FIG. 6C

710 — INTRODUCE FLUID SAMPLE TO ANALYTICAL TEST STRIP

700 —

720 — APPLY VOLTAGE SIGNAL ACROSS STRIP AND MEASURE CURRENT SIGNAL

730 — SUCCESSIVELY PRODUCE IN-PHASE AND QUADRATURE SIGNALS

740 — DETERMINE PHASE SHIFT CORRESPONDING TO FLUID SAMPLE

750 — DETERMINE HEMATOCRIT VALUE OF WHOLE BLOOD SAMPLE

760 — DETERMINE GLUCOSE VALUE OF WHOLE BLOOD SAMPLE USING HEMATOCRIT VALUE

**FIG. 7**

730 — PRODUCE SIGNALS

810 — PROVIDE RESULTANT VOLTAGE SIGNAL

820 — PROVIDE FIRST REFERENCE SIGNAL

830 — PROVIDE SECOND REFERENCE SIGNAL

**FIG. 8**

720 — APPLY VOLTAGE

910 — APPLY FIRST-FREQUENCY SIGNAL

920 — APPLY SECOND-FREQUENCY SIGNAL AND MEASURE

930 — SUCCESSIVELY PRODUCE SECOND SIGNALS

**FIG. 9**

1020

F-DRV = sin(ωt)

1030

ΔΦ

1040

TIA-OUT =sin(ωt+Φ)

1000

REF0=sin(ωt)

1090

REF90=sin(ωt+90°)

1050

1060

×

1070

LPF

VOUT

**FIG. 10**

Europäisches
Patentamt

European
Patent Office

Office européen
des brevets

# EUROPEAN SEARCH REPORT

**Application Number**

EP 14 17 1004

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/084589 A1 (KRAFT ULRICH [DE] ET AL) 4 April 2013 (2013-04-04)<br>* claims 1,14 *<br>* abstract *<br>* figures *<br>* paragraphs [0013] - [0044] *<br>----- | 1-23 | INV.<br>G01N27/327<br>G01N33/487<br>A61B5/145 |
| A | YUXIANG YANG ET AL: "Design and preliminary evaluation of a portable device for the measurement of bioimpedance spectroscopy; Design and preliminary evaluation of a portable device", PHYSIOLOGICAL MEASUREMENT, INSTITUTE OF PHYSICS PUBLISHING, BRISTOL, GB, vol. 27, no. 12, 1 December 2006 (2006-12-01), pages 1293-1310, XP020105705, ISSN: 0967-3334, DOI: 10.1088/0967-3334/27/12/004<br>* the whole document *<br>----- | 1-23 | |
| A | US 5 642 734 A (RUBEN PAUL [US] ET AL) 1 July 1997 (1997-07-01)<br>* abstract *<br>* figures *<br>----- | 1-23 | TECHNICAL FIELDS SEARCHED (IPC)<br><br>G01N<br>A61B<br>G09G |
| A,D | US 2007/084734 A1 (ROBERTS NEIL [US] ET AL) 19 April 2007 (2007-04-19)<br>* abstract *<br>----- | 1-23 | |
| A,D | US 2007/087397 A1 (KRAFT ULRICH [DE] ET AL) 19 April 2007 (2007-04-19)<br>* abstract *<br>----- | 1-23 | |
| A,D | WO 2010/049669 A1 (LIFESCAN SCOTLAND LTD [GB]; GUTHRIE BRIAN [GB]; MACRAE ALLAN [GB]; LAW) 6 May 2010 (2010-05-06)<br>* abstract *<br>----- | 1-23 | |

The present search report has been drawn up for all claims

| Place of search | Date of completion of the search | Examiner |
|---|---|---|
| Munich | 27 October 2014 | Ruchaud, Nicolas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT**
**ON EUROPEAN PATENT APPLICATION NO.** EP 14 17 1004

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2014

| Patent document cited in search report | Publication date | Patent family member(s) | Publication date |
|---|---|---|---|
| US 2013084589 A1 | 04-04-2013 | AU 2012314100 A1 | 02-05-2013 |
| | | CA 2850135 A1 | 04-04-2013 |
| | | CN 103842808 A | 04-06-2014 |
| | | EP 2766720 A1 | 20-08-2014 |
| | | KR 20140069259 A | 09-06-2014 |
| | | US 2013084589 A1 | 04-04-2013 |
| | | WO 2013045950 A1 | 04-04-2013 |
| US 5642734 A | 01-07-1997 | NONE | |
| US 2007084734 A1 | 19-04-2007 | CN 1991355 A | 04-07-2007 |
| | | CN 102520045 A | 27-06-2012 |
| | | EP 1783486 A1 | 09-05-2007 |
| | | EP 2261651 A1 | 15-12-2010 |
| | | JP 4859619 B2 | 25-01-2012 |
| | | JP 2007114197 A | 10-05-2007 |
| | | US 2007084734 A1 | 19-04-2007 |
| | | US 2011174616 A1 | 21-07-2011 |
| US 2007087397 A1 | 19-04-2007 | CN 1996000 A | 11-07-2007 |
| | | CN 102520029 A | 27-06-2012 |
| | | EP 1775587 A2 | 18-04-2007 |
| | | EP 2498083 A1 | 12-09-2012 |
| | | ES 2438220 T3 | 16-01-2014 |
| | | HK 1103531 A1 | 27-06-2014 |
| | | JP 4914170 B2 | 11-04-2012 |
| | | JP 5080671 B2 | 21-11-2012 |
| | | JP 2007114198 A | 10-05-2007 |
| | | JP 2011232360 A | 17-11-2011 |
| | | US 2007087397 A1 | 19-04-2007 |
| | | US 2009322341 A1 | 31-12-2009 |
| WO 2010049669 A1 | 06-05-2010 | AU 2009309458 A1 | 06-05-2010 |
| | | CA 2741822 A1 | 06-05-2010 |
| | | CN 102265330 A | 30-11-2011 |
| | | EP 2361427 A1 | 31-08-2011 |
| | | JP 5421382 B2 | 19-02-2014 |
| | | JP 2012507056 A | 22-03-2012 |
| | | KR 20110079743 A | 07-07-2011 |
| | | US 2011210951 A1 | 01-09-2011 |
| | | WO 2010049669 A1 | 06-05-2010 |

EPO FORM P0459

For more details about this annex : see Official Journal of the European Patent Office, No. 12/82

**EP 2 811 290 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070084734 A
- US 20070087397 A
- WO 2010049669 A